# EUROPÄISCHE PATENTSCHRIFT

(11) **EP 2 498 834 B1**
(45) Veröffentlichungstag und Bekanntmachung des Hinweises auf die Patenterteilung: **16.10.2013**
(21) Anmeldenummer: 10785011.7
(22) Anmeldetag: 12.11.2010
(51) Int. Cl.: A61L 29/16, A61L 29/04

(54) **VERWENDUNG VON POLYOXYALKYLENDIAMIN-BASIERTEN POLYGUANIDINDERIVATEN FÜR MEDIZINISCHE ARTIKEL**
USE OF POLYOXYALKYLENE DIAMINE-BASED POLYGUANIDINE DERIVATIVES FOR MEDICAL ARTICLES
UTILISATION DE DÉRIVÉS DE POLYGUANIDINE À BASE DE POLYOXYALKYLÈNE DIAMINE POUR DES ARTICLES MÉDICAUX

(30) Priorität: 12.11.2009 DE 102009052725
(43) Veröffentlichungstag der Anmeldung: 19.09.2012
(73) Patentinhaber: B. Braun Melsungen AG, 34212 Melsungen (DE)
(72) Erfinder: RIEMANN, Thomas, 37247 Großalmerode (DE); WEIß, André, 34302 Guxhagen (DE); SCHMITT, Jürgen, 35274 Kirchhain (DE); BRETHAUER, Ulrich, 34327 Körle (DE); SIPPEL, Martin, 34212 Melsungen (DE)
(74) Vertreter: von Kreisler Selting Werner
(86) Internationale Anmeldenummer: PCT/EP2010/067406
(87) Internationale Veröffentlichungsnummer: WO 2011/058145

(56) Entgegenhaltungen:
- EP-A2- 2 305 322
- WO-A2-2009/009814
- WO-A2-2009/080239
- DE-A1-102004 011 293
- US-A1- 2002 120 333

## Beschreibung

Die Erfindung betrifft die Verwendung polymerer Guanidinderivate mit biozider Wirkung in einer Zusammensetzung für medizinische Artikel.

Darüber hinaus betrifft die Erfindung ein Verfahren zur Herstellung eines medizinischen Artikels.

Medizinische Artikel bzw. Gegenstände, die in den Körper eines Patienten eingeführt werden, beispielsweise intravasale Katheter, Beatmungsschläuche oder Stents müssen eine möglichst glatte Oberfläche haben, um Beschwerden beim Patienten als auch Ablagerungen auf den Oberflächen zu minimieren. Hergestellt werden diese medizinischen Artikel sowie deren Verpackung oft durch Verfahren der Kunststofftechnik, beispielsweise Formpress-, Spritzguss-, Tiefzieh- und Extrusionsverfahren aus einem Kunststoff, wobei versucht wird, möglichst glatte Oberflächen zu erzielen.

Zur Vermeidung von Infektionen ist es vorteilhaft die medizinischen Artikel mit antimikrobiell wirkenden Mitteln ausgerüstet sein. An die biozide Ausrüstung der medizinischen Artikel werden hohe Anforderungen gestellt, da die Artikel mit Körpergewebe und Körperflüssigkeit in Kontakt kommen. Beispielsweise stellen Katheter, die durch die Hautoberfläche in Arterien oder Venen eingeführt werden, aber auch Wund- oder Thoraxdränageschläuche, häufige Infektionsquellen dar. Insbesondere besteht bei Patienten, die Langzeit-Urinkatheter benötigen, die Gefahr von Harnwegsinfektionen, die zu einer Bakterien- oder einer chronischen Pyelonephritis führen können.

Im medizinischen Bereich spielen insbesondere die zentralen Venenkatheter eine zunehmend größere Rolle bei medizinischen Behandlungen und chirurgischen Eingriffen. Zentrale Venenkatheter werden immer häufiger im Rahmen der Intensivmedizin, aber auch bei Anwendungen, z.B. bei Knochenmarks- und Organtransplantationen, Hämodialyse oder Cardiothoraxchirurgie, eingesetzt.

Ein ähnliches Infektionsrisiko ist aber auch bei allen Vorrichtungen gegeben, welche die Katheter beispielsweise mit Infusionsbehältnissen außerhalb des Körpers verbinder, beispielsweise Verbindungsstücke, T-Stücke, Kupplungen, Filter, Überleitungssysteme, Ventile, Spritzen und Mehrweg-Hähne. All diese Gegenstände werden für die Zwecke dieser Beschreibung und der Patentansprüche als "medizinische Artikel" bezeichnet.

Für die medizinischen Artikel, insbesondere die Katheter, müssen aber nicht nur die hohen Anforderungen an eine glatte Oberfläche beispielsweise zur Vermeidung bzw. Verringerung von Thrombozytenapposition und Biofilmbildung und an die biozide Ausrüstung, die ein Wachsen von Mikroben auf der Oberfläche verhindern oder die Mikroben ganz abtöten sollen, gewährleistet sein, sondern es muss auch sichergestellt werden, dass die biozide Ausrüstung der medizinischen Gegenstände die Materialeigenschaften der medizinischen Artikel nicht negativ beeinflusst. Darüber hinaus muss gewährleistet werden, dass die medizinischen Artikel, insbesondere dann, wenn sie mit Flüssigkeit in Kontakt kommen, einerseits eine hohe biozide Wirksamkeit aufweisen, aber andererseits auch nicht an die Flüssigkeit abgegeben werden, um eine Anreicherung der bioziden Wirkstoffe im Körper zu vermeiden. Die Abgabe eines bioziden Wirkstoffs aus einem medizinischen Artikel bei Flüssigkeitskontakt wird auch als "Leaching" bezeichnet.

Aus der EP 0 229 862 sind medizinische Artikel aus Polyurethan bekannt, auf deren Oberfläche ein antimikrobielles Mittel aufgebracht ist. Aus der EP 0 379 269 und aus der DE 10 2004 011 293 sind ebenfalls medizinische Artikel, insbesondere Schläuche, bekannt, welche aus einem thermoplastischen Polymer geformt sind und als antimikrobiell wirkendes Mittel Chlorhexidin enthalten. Diese Artikel werden hergestellt, indem zunächst eine Mischung aus Chlorhexidin und Kunststoffgranulate eines thermoplastischen Polymers bereitgestellt wird, die zu einer Schmelze verarbeitet wird, in der das Chlorhexidin gleichmäßig verteilt ist, wobei die Schmelze durch eine Matrix extrudiert wird, um den medizinischen Artikel zu formen. Der Einsatz von bioziden Mitteln auf Basis von Biguaniden, wie beispielsweise Chlorhexidin oder Polyhexamethylenbiguanid, ist jedoch nicht immer zufriedenstellend. Insbesondere besteht ein Verbesserungsbedarf im Hinblick auf die Glattheit der Oberfläche der medizinischen Artikel als auch im Hinblick auf die Reduzierung oder Steuerung des "Leaching"-Effekts. Darüber hinaus war es Aufgabe der vorliegenden Erfindung, medizinische Artikel zur Verfügung zu stellen, die mit bioziden Wirkstoffen ausgerüstet sind, die auch im Hinblick auf eine ResistenzEntwicklung der Bakterienstämme bezüglich herkömmlicher antimikrobieller Mittel hochwirksam sind. Die Aufgabe der vorliegenden Erfindung wird durch die Verwendung polymerer Guanidinderivate als Additive zu Werkstoffen für medizinische Artikel gelöst.

Gegenstand der vorliegenden Erfindung ist daher die Verwendung wenigstens eines polymeren Guanidinderivats mit biozider Wirkung, welches erhältlich ist durch Polykondensation eines Guanidin-Säureadditionssalzes mit einem Amingemisch, das wenigstens ein Diamin enthält, welches ausgewählt ist aus der Gruppe bestehend aus Alkylendiamin und Oxyalkylendiamin, als Additiv in einer Zusammensetzung für schlauchförmige medizinische Artikel.

Es hat sich gezeigt, dass bei Verwendung der genannten polymeren Guanidinderivate in Zusammensetzungen, die Kunststoffe, insbesondere thermoplastische Polymere, enthalten, wobei die Kunststoffe für die Herstellung medizinischer Artikel eingesetzt werden, sehr glatte Oberflächen des Kunststoffs gewährleistet werden können, welche sogar jene des Kunststoffs ohne den Zusatz der polymeren Guanidinderivate übertrifft. Neben der hervorragenden bioziden Wirksamkeit, die die polymeren Guanidinderivate den Zusammensetzungen für die medizinischen Artikel verleihen, hat sich auch überraschend herausgestellt, dass die polymeren Guanidinderivate, den Zusammensetzungen, ein gut steuerbares Freisetzungsverhalten ("Leaching"-Effekt) aufweisen. Die Steuerbarkeit reicht von keiner Freisetzung bis zu Freisetzungsraten von mehreren mg/m²/h.

Die erfindungsgemäß verwendeten polymeren Guanidinderivate sowie deren Herstellung sind dem Fachmann bekannt und beispielsweise in der WO-A1-01/85676 sowie in der WO-A1-06/047800 beschrieben.

Die erfindungsgemäß verwendeten polymeren Guanidinderivate können sowohl als Homopolymer als auch als Copolymer vorliegen. Vorteilhaft ist dabei, wenn das Guanidin-Säureadditionssalz Guanidiniumhydrochlorid ist. Es sind aber auch weitere Guanidin-Säureadditionssalze, auf Basis anorganischer oder organischer Säuren ebenfalls geeignet. Beispielsweise die Hydroxide, Hydrogensulfate sowie Acetate. Besonders geeignete und wirksame polymere Guanidinderivate liegen in Form ihrer Hydroxid-Salze vor. Diese können beispielsweise durch Anionenaustausch aus den korrespondierenden Chlorid-Salzen erhalten werden.

Die polymeren Guanidinderivate mit biozider Wirkung sind erhältlich durch Polykondensation eines Guanidin-Säureadditionssalzes mit einem Amingemisch, das wenigstens ein Diamin enthält, welches ausgewählt ist aus der Gruppe bestehend aus Aklylendiamin und Oxyalkylendiamin.

Im Rahmen der vorliegenden Erfindung wird der Begriff polymeres Guanidinderivat für Guanidinderivate verwendet in denen 2 oder mehr Repetitionseinheiten auftreten. Der Begriff "Polymer" erfasst somit auch bereits Dimere, Trimere oder beispielsweise Oligomere.

Bevorzugt umfasst das Amingemisch ein Alkylendiamin, das besonders bevorzugt eine Verbindung der allgemeinen Formel

NH₂(CH₂)ₙNH₂

in welcher n eine ganze Zahl zwischen 2 und 10, bevorzugt 4 oder 6, ist. Bevorzugt einzusetzende Alkylendiamine tragen endständige Aminogruppen. Speziell bevorzugt ist das Hexamethylendiamin (Hexan-1,6-diamin). Das Alkylendiamin kann in der Polykondensationsreaktion in Mischung mit weiteren Polyaminen, beispielsweise Di- und/oder Triaminen unter Ausbildung von Copolymeren eingesetzt werden.

Vorzugsweise umfasst das Amingemisch wenigstens ein Oxyalkylendiamin.

Als Oxyalkylendiamine eignen sich insbesondere solche Oxyalkylendiamine, die endständige Aminogruppen aufweisen. Ein bevorzugtes Oxyalkylendiamin ist eine Verbindung der allgemeinen Formel

NH₂[(CH₂)₂O)]ₙ(CH₂)₂NH₂

in welcher n eine ganze Zahl zwischen 2 und 6, bevorzugt zwischen 2 und 5, weiter bevorzugt zwischen 2 und 4 und insbesondere 2 ist. Bevorzugt sind Oxyethylendiamine, im Speziellen Diethylenglycoldiamin oder Triethylenglycoldiamin. Weiter bevorzugt eingesetzt werden können Polyoxypropylendiamine, insbesondere Di- oder Tripropylenglycoldiamin.

Bevorzugt liegt das polymere Guanidinderivat als Homopolmyer vor. In diesen Fällen besteht das Amingemisch aus dem Alkylendiamin oder aus einem Oxyalkylendiamin.

In einer weiteren bevorzugten Ausführungsform besteht das Amingemisch aus dem Alkylendiamin Hexamethylendiamin (Hexan-1,6-diamin). In dieser Variante besteht das polymere Guanidinderivat somit aus einem Homopolymer, beispielsweise das Poly-(hexamethylen-guanidinium-chlorid) (PHMGC).

In einer weiteren bevorzugten Ausführungsform besteht das Amingemisch aus dem Oxyalkylendiamin Triethylenglycoldiamin. Durch Polykondensation mit einem Guanidinsäureadditionssalz entsteht daraus beispielsweise das Homopolymer Poly-[2-(2-ethoxy)-ethoxyethyl)-guanidinium-chlorid].

In einer weiteren bevorzugten Ausführungsform liegen die erfindungsgemäß verwendeten polymeren Guanidinderivate in Form von Copolymeren vor. Diese können dabei sowohl willkürlich gemischt als auch als Blockcopolymere vorliegen. Im Falle von Copolymeren enthält das Amingemisch wenigstens zwei verschiedene Amine. Das Amingemisch enthält dabei eine erste Komponente und wenigstens eine zweite Komponente, wobei
- die erste Komponente ein Diamin ist, ausgewählt aus der Gruppe bestehend aus Alkylendiamin und Oxyalkylendiamin, und wobei
- die zweite Komponente ein Diamin ist, das ausgewählt aus der Gruppe bestehend aus Alkylendiamin und Oxyalkylendiamin, und
wobei die erste Komponente von der zweiten Komponente verschieden ist.

Als besonders geeignete copolymere Guanidinderivate haben sich solche herausgestellt, bei denen die erste Komponente Alkylendiamin und die zweite Komponente ein Oxyalkylendiamin ist. Besonders bevorzugt sind copolymere Guanidinderivate bei denen im Amingemisch die erste Komponente Hexamethylendiamin und die zweite Komponente Triethylenglycoldiamin ist.

Bei der Herstellung von Copolymeren kann das Mischungsverhältnis der einzusetzenden Amine in weiten Bereichen variiert werden. Bevorzugt sind allerdings copolymere Guanidinderivate, bei denen die Monomere des Amingemischs, also die erste Komponente und die zweite Komponente, in einem Molverhältnis von 4 : 1 bis 1 : 4, vorzugsweise 2 : 1 bis 1 : 2, vorliegt.

Die erfindungsgemäß einzusetzenden polymeren Guanidinderivate besitzen bevorzugt ein mittleres Molekulargewicht (Gewichtsmittel) im Bereich von 500 bis 7000, insbesondere von 1000 bis 5000 Dalton.

In einer weiteren bevorzugten Ausführungsform der vorliegenden Erfindung liegt das erfindungsgemäß einzusetzende polymere Guanidinderivat als Mischung aus mindestens 2 unterschiedlichen polymeren Guanidinderivaten vor. In einer speziellen Ausführungsform umfasst die Mischung der polymeren Guanidinderivate sowohl ein erstes Homopolymer basierend auf einem Alkylendiamin, vorzugsweise Poly-(hexamethylen-guanidinium-chlorid) als auch ein zweites Homopolymer basierend auf einem Oxyalkylendiamin, beispielsweise Poly-[2-(2-ethoxy)-ethoxyethyl)-guanidinium-chlorid].

In einer bevorzugten Ausführungsform umfasst das polymere Guanidinderivat das erste Homopolymer und das zweite Homopolymer in einem Gewichtsverhältnis von 5:1 bis 1:5, vorzugsweise 1:1 bis 1:4 und insbesondere 1:2 bis 1:4. In einer besonders bevorzugten Ausführungsform umfasst das polymere Guanidingemisch Poly-(hexamethylen-guanidinium-chlorid) (erstes Homopolymer) und Poly-[2-(2-ethoxy)-ethoxyethyl)-guanidinium-chlorid] (zweites Homopolymer) in einem Gewichtsverhältnis (erstes Homopolymer zu zweitem Homopolymer) von 1:1 bis 1:5, vorzugsweise 1:2 bis 1:4, insbesondere 1:3. Solche Mischungen zeigen eine hervorragende Eigenschaft bezüglich ihrer Verarbeitbarkeit mit Kunststoffen, die zu medizinischen Artikeln verarbeitet werden.

Insbesondere bezüglich der bioziden Aktivität und des "Leaching"-Verhaltens bei der Einarbeitung der polymeren Guanidinderivate in thermoplastische Polymere, die dann zu medizinischen Artikeln verarbeitet werden, haben sich die homopolymeren Guanidinderivate auf Basis von Oxyalkylendiaminen, insbesondere das Poly-[2-(2-ethoxy)-ethoxyethyl)-guanidinium-chlorid], als besonders geeignet herausgestellt.

Die erfindungsgemäß verwendeten polymeren Guanidine weisen allesamt eine antibakterielle Wirkung auf, die sich mit Hilfe der sogenannten "minimalen Hemmkonzentration" beschreiben lässt. Diese gibt die niedrigste Bakterizidkonzentration an, die das Wachstum von Bakterien in einer bestimmten Lösung hemmt. Zur Überprüfung der bioziden Wirkung der erfindungsgemäß einzusetzenden polymeren Guanidinderivate werden diese Verbindungen, in einem Bakterien-Nährmedium, vorzugsweise Tryptic Soy Broth, angesetzt und auf verschiedene Konzentrationen verdünnt. Diese Lösungen unterschiedlicher Konzentration werden mit einer Suspension von *Escherichia coli* inokuliert und für 24 h bei 37°C inkubiert.

Unter der minimalen Hemmkonzentration (MHK) wird dann die geringste Konzentration des zu untersuchenden Polyguanidins in der Lösung verstanden, bei der das Wachstum der Bakterien gehemmt wird. Bei der entsprechenden Lösung ist dann keine Eintrübung durch das Wachstum der Bakterien beobachtbar. Besonders günstig ist dabei eine minimale Hemmkonzentration von weniger als 50 µg/ml. Bevorzugt weisen die erfindungsgemäß zu verwendenden polymeren Guanidinderivate eine minimale Hemmkonzentration von weniger als 10 µg/ml auf. Je geringer diese Konzentration ist, umso effektiver kann das entsprechende polymere Guanidinderivat als Biozid eingesetzt werden.

In einer bevorzugten Ausführungsform weisen die erfindungsgemäß einzusetzenden polymeren Guanidinderivate eine minimale Hemmkonzentration von 50 µg/ml oder weniger, bevorzugt 30 µg/ml oder weniger, besonders bevorzugt 10 µg/ml oder weniger auf.

Die erfindungsgemäß einzusetzenden polymeren Guanidinderivate lassen sich relativ einfach herstellen. Die Polykondensation kann durch Mischen eines Äquivalents eines Guanidinsäureadditionssalzes mit einem Äquivalent der Aminmischung und anschließendes Erhitzen, vorzugsweise in einem Bereich von 140 bis 180 °C und Rühren der Schmelze bei erhöhten Temperaturen, vorzugsweise in einem Bereich von 140 bis 180 °C, bis die Gasentwicklung beendet ist, erfolgen. Die Polykondensation erfolgt regelmäßig in einem Zeitraum von mehreren Stunden, bei der die Schmelze bevorzugt in dem Temperaturbereich von 140 bis 180 °C gerührt wird. Ein bevorzugter Reaktionszeitraum ist 1 bis 15 Stunden, vorzugsweise 5 bis 10 Stunden.

Erfindungsgemäß werden die polymeren Guanidinderivate als Additive in Zusammensetzungen für medizinische Artikel eingesetzt. Je nach biozider Wirksamkeit der polymeren Guanidinderivate sowie der Art und des Aufbaus des medizinischen Artikels können die Zusammensetzungen für den medizinischen Artikel in einer bevorzugten Ausführungsform das polymere Guanidinderivat in einer Menge von maximal 10,0 Gew.-%, insbesondere von 0,01 bis 6 Gew.-% und im Speziellen in einer Menge von 1,0 bis 4,0 Gew.-%, jeweils bezogen auf die Zusammensetzung für den medizinischen Artikel, enthalten.

Ein besonderer Vorteil der erfindungsgemäß einzusetzenden polymeren Guanidinderivate ist deren Einarbeitbarkeit in Kunststoffen, insbesondere thermoplastischen Polymeren, die häufig den wesentlichen Bestandteil von Zusammensetzungen für medizinische Artikel bilden. Es hat sich überraschend gezeigt, dass sich die polymeren Guandinderivate nicht nur problemlos in Kunstoffen, insbesondere thermoplastischen Polymerzusammensetzungen einarbeiten lassen, sondern dass darüber hinaus die mechanischen Eigenschaften, wie beispielsweise die Zugbelastbarkeit oder der Biegewiderstand, nur unwesentlich beeinflusst werden. Darüber hinaus zeigte sich überraschend, dass der Einsatz der polymeren Guanidinderivate in Zusammensetzungen umfassend thermoplastische Polymere für medizinische Artikel bei der Verarbeitung zu extrem glatten Oberflächen führt und darüber hinaus keinen "Leaching"-Effekt aufweist, d.h., die bioziden polymeren Guanidinderivate werden nicht von Flüssigkeiten wie beispielsweise Wasser oder Ethanol aus dem Polymer-Blend freigesetzt. Gleichwohl weisen die mit den polymeren Guanidinderivaten ausgerüsteten Zusammensetzungen, insbesondere Zusammensetzungen umfassend thermoplastische Polymere für medizinische Artikel, eine hervorragende antimikrobielle Wirksamkeit auf.

In einer bevorzugten Ausführungsform umfasst die Zusammensetzung für medizinische Artikel weiterhin thermoplastische Polymere, insbesondere ausgewählt aus Polyurethan, Polyolefin, Polyvinylchlorid, Polycarbonat, Polystyrol, Polyethersulfon, Silikon und Polyamid. Besonders bevorzugt umfassen die Zusammensetzungen für medizinische Artikel Polymere ausgewählt aus Polyurethan oder Polyethylen oder Polypropylen.

Es hat sich zudem überraschend gezeigt, dass die erfindungsgemäß einzusetzenden polymeren Guanidinderivate auch kovalent mit der Kunststoffmatrix, zu der sie additiviert werden, verknüpft werden können. Die kovalente Anbindung der polymeren Guanidinderivate kann beispielsweise durch reactive processing bei erhöhten Temperaturen, beispielsweise oberhalb von 100°C, vorzugsweise oberhalb von 140°C in Gegenwart geeigneter thermoplastischer Polymere erfolgen.

Es hat sich gezeigt, dass Zusammensetzungen, die die polymeren Guanidinderivate kovalent angebunden an den Kunststoff aufweisen, weiter verbesserte Eigenschaften bezüglich der Glätte der schlauchförmigen medizinischen Artikel als auch ein weiter verringertes Leaching-Verhalten aufweisen.

Die kovalent mit Kunststoffen, vorzugsweise thermoplastischen Polymeren verknüpften polymeren Guanidinderivate, zeigen zudem überraschend eine verbesserte Biokompatibilität, was diese Produkte besonders für medizinische Artikel eignet, die mit Körperflüssigkeiten in Berührung kommen, z.B. Katheter.

In einer bevorzugten Ausführungsform liegen die polymeren Guanidinderivate daher kovalent verknüpft mit dem Kunststoff vor.

Bevorzugt liegen wenigstens 50 Gew.-%, noch mehr bevorzugt wenigstens 90 Gew.-% der erfindungsgemäßen additivierten polymeren Guanidinderivate kovalent verknüpft mit dem Kunststoff vor.

Darüber hinaus können die Zusammensetzungen für medizinische Artikel weitere übliche Zusatzstoffe enthalten. Hier kommen insbesondere unter physiologischen Bedingungen inerte Füllstoffe in Frage. Besonders geeignet ist Bariumsulfat. Beispielsweise kann ein geeignetes BaSO₄ unter dem Handelsnamen Blancfix^{®} von der Firma Sachtleben Chemie GmbH bezogen werden. Die Füllstoffe liegen bevorzugt in den Zusammensetzungen für medizinische Artikel in einer Menge von 10 bis 35 Gew.-%, bezogen auf die Gesamtmischung, vor. Vorteilhafterweise weisen die Füllstoffe eine mittlere Teilchengröße von 0,01 µm bis 10 µm auf.

In einer bevorzugten Ausgestaltung der vorliegenden Erfindung ist die Zusammensetzung jedoch im Wesentlichen frei von silikatischen Füllstoffen, da diese die Oberflächenglätte als auch den Leachingeffekt negativ beeinflussen können.

Im Wesentlichen frei im Rahmen der vorliegenden Erfindung bedeutet, dass die silikatischen Füllstoffe in einer Menge unterhalb von 1 Gew.-%, bevorzugt unterhalb von 0,5 Gew.-%, weiter bevorzugt unterhalb von 0,01 Gew.-% und insbesondere frei von jeglichen silikatischen Füllstoffen, vorliegen können, wobei sich die Gewichtsangaben auf das Gesamtgewicht der Zusammensetzung für die Herstellung des schlauchförmigen medizinischen Artikels bezieht.

Schlauchförmige medizinische Artikel im Sinne der vorliegenden Erfindung sind solche medizinischen Artikel, die Fluide führen können. Insbesondere sind die medizinischen Artikel ausgewählt aus der Gruppe bestehend aus Katheder; zentralvenösen Kathetern; peripheren Venenkathetern; Beatmungsschläuche; Stents; Kuppelungen; Ports; Überleitungssysteme; Konnektoren, Spikes, Ventile, Dreiwegehähne, Spritzen, Leitungen, Zuspritzports; Wunddrainage; Thoraxdrainagen und Sonden.

Besonders bevorzugte schlauchförmige medizinische Artikel sind Katheter, insbesondere solche, die durch Extrusion von Zusammensetzungen umfassend Polyurethan und/oder Polyethylen, hergestellt werden.

Ein weiterer Gegenstand der vorliegenden Erfindung ist ein Verfahren zur Herstellung eines schlauchförmigen medizinischen Artikels, umfassend die folgenden Schritte:
a) Zusammenführen und Vermischen eines polymeren Guanidinderivats mit biozider Wirkung, welches erhältlich ist durch Polykondensation eines Guanidin-Säureadditionssalzes mit einem Amingemisch, das wenigstens ein Diamin enthält, welches ausgewählt ist aus der Gruppe bestehend aus Aklylendiamin und Oxyalkylendiamin, mit mindestens einem Kunststoff, vorzugsweise einem thermoplastischen Polymer,
b) Einsetzen des unter a) erhaltenen Gemisches in einem oder mehreren Formgebungsverfahren unter Ausbildung eines schlauchförmigen medizinischen Artikels.

Bevorzugte polymere Guanidinderivate und bevorzugte Kunststoffe entsprechen den zuvor genannten.

Das Vermischen in Schritt a) erfolgt bevorzugt durch Schmelzkneten. Das polymere Guanidinderivat kann als wässrige Lösung dem geschmolzenen Kunststoff zugegeben werden und anschließend in einem Extruder vermischt werden. Bevorzugt erfolgt das Schmelzkneten bei Temperaturen oberhalb von 100°C, weiter bevorzugt oberhalb von 150°C.

Bevorzugt wird das polymere Guanidinderivat dem Formgebungsverfahren in Schritt b) als Granulat oder Masterbatch zugeführt. Die Herstellung von Granulaten kann nach dem Fachmann auf dem Gebiet der Kunststofftechnologie geläufigen Verfahren erfolgen. Bevorzugt ist der Masterbatch ein Granulat, das das polymere Guanidinderivat in einer höheren Konzentration als der vorgesehenen Endkonzentration im medizinischen Artikel aufweist. Bei Einsatz eines Masterbatches wird dieser daher durch Zugabe von weiterem Kunststoff weiter auf die gewünschte Endkonzentration verdünnt.

Das in Schritt b) des erfindungsgemäßen Verfahrens zum Einsatz kommende Formgebungsverfahren ist bevorzugt ein Extrusionsverfahren. Hiermit können beispielsweise schlauchartige Komponenten des Katheters hergestellt werden.

Die Formgebungsverfahren werden vorzugsweise bei Temperaturen oberhalb des Schmelzpunkts des in Schritt a) hergestellten Gemisches durchgeführt, besonders bevorzugt in einem Temperaturbereich oberhalb von 160 °C, weiter bevorzugt in einem Bereich von 180 - 260 °C.

Als Ergebnis entsteht ein Werkstoff mit antimikrobiellen Eigenschaften bei welchem das Additiv (polymeres Guanidinderivat) physikalisch eingemischt oder ggf. chemisch an den jeweiligen Kunststoff gebunden ist.

Ein weiterer Gegenstand der vorliegenden Erfindung ist ein schlauchförmiger medizinischer Artikel umfassend ein polymeres Guanidinderivat mit biozider Wirkung, welches erhältlich ist durch Polykondensation eines Guanidin-Säureadditionssalzes mit einem Amingemisch, das wenigstens ein Diamin enthält, welches ausgewählt ist aus der Gruppe bestehend aus Aklylendiamin und Oxyalkylendiamin, sowie mindestens einen Kunststoff, insbesondere ein thermoplastisches Polymer.

Bevorzugte polymere Guanidinderivate und bevorzugte Kunststoffe entsprechen den zuvor genannten.

Das in dem erfindungsgemäßen medizinischen Artikel sowie in dem erfindungsgemäßen Verfahren einzusetzende thermoplastische Polymer ist bevorzugt ein thermoplastisches Polyurethan. Es haben sich hier als besonders geeignet Polyurethane herausgestellt, die aus einer Kombination von 4,4'-Diphenylmethan-diisocyanat (MDI) und einem Polyol auf Polyester- oder Polyetherbasis erhältlich sind. Vorteilhafterweise umfasst das Polyol einen Polytetramethylenglykolether. Weitere geeignete thermoplastische Polymere, die bevorzugt für Zusammensetzungen der erfindungsgemäßen medizinischen Artikel in Frage kommen, sind beispielsweise ausgewählt aus Polyurethan, Polyolefin, Polyvinylchlorid, Polycarbonat, Polystyrol, Polyethersulfon, Silikon und Polyamid. Besonders bevorzugt umfassen die Zusammensetzungen für medizinische Artikel Polyurethan oder Polyethylen oder Polypropylen oder Polyamid.

Ein besonders geeignetes Polyamid ist unter dem Handelsnamen Pebax^{®} (Arkema) erhältlich. Es handelt sich dabei um ein Polyamid, das Polyether-Blöcke aufweist.

Bevorzugte medizinische Artikel entsprechen den zuvor genannten. Insbesondere bevorzugte medizinische Artikel sind Katheter.

Ein weiterer Gegenstand der vorliegenden Erfindung ist ein biozides Material, umfassend ein thermoplastisches Polymer, das mit wenigstens einem polymeren Guanidinderivat, welches erhältlich ist durch Polykondensation eines Guanidin-Säureadditionssalzes mit einem Amingemisch, das wenigstens ein Diamin enthält, welches ausgewählt ist aus der Gruppe bestehend aus Alkylendiamin und Oxalkylendiamin, kovalent verknüpft ist.

Bevorzugte Ausführungsformen des polymeren Guanidinderivats sowie des thermoplastischen Polymers wurden bereits zuvor beschrieben. Für das biozide Material gemäß der vorliegenden Erfindung eignen sich insbesondere thermoplastische Polymere, die ausgewählt sind aus der Gruppe der Polyurethane, Polyester, Polyamide, Polycarbonate, Polyharnstoffe, Polyesteramide und insbesondere Polykondensate.

Bei der Herstellung der erfindungsgemäßen bioziden Materialien erfolgt die Verknüpfung des polymeren Guanidinderivats mit dem thermoplastischen Polymer bevorzugt unter den Bedingungen des reactive processing. Hierzu wird das polymere Guanidinderivat mit dem thermoplastischen Polymer vermischt und die Reaktionsbedingungen so gewählt, dass eine kovalente Anbindung des polymeren Guanidinderivats an das thermoplastische Polymer erfolgt. Dies kann beispielsweise dadurch geschehen, dass das thermoplastische Polymer so ausgewählt wird, dass es noch Reaktivgruppen trägt, beispielsweise Isocyanatgruppen.

Die Anbindung des polymeren Guanidinderivats kann jedoch auch unter ausgewählten Bedingungen im Rahmen einer Umkondensation erfolgen. Besonders geeignete thermoplastische Polymere sind hierfür aliphatische Polyurethane und/oder aromatische Polyurethane.

Es hat sich überraschend herausgestellt, dass das biozide Material der vorliegenden Erfindung insbesondere dann erhältlich ist, wenn das thermoplastische Polymer bei Temperaturen oberhalb von 120°C, bevorzugt oberhalb von 160°C schmelzextrudiert wird und eine wässrige Lösung, vorzugsweise eine 20 bis 50 Gew.-%ige wässrige Lösung des polymeren Guanidinderivats zugegeben wird. Gerade unter diesen Reaktionsbedingungen konnte die kovalente Anbindung der polymeren Guanidinderivate an das thermoplastische Polymer beobachtet werden.

Besonders geeignet für die kovalente Anbindung, insbesondere im Rahmen einer Schmelzextrusion, sind Polyester, Polylactide, Polycaprolactone, Polyamide, Polyesteramide, Polycarbonate, Polyurethane und Polyharnstoffe.

Besonders bevorzugt sind Polyethylenterephthalat, Polybutylenterephthalat, Polyamid PA6, PA66, PA610, PA11, PA12, aliphatische und aromatische Polycarbonate sowie aliphatische und aromatische Polyurethane.

Das erfindungsgemäße biozide Material lässt sich hervorragend verarbeiten, zeigt insbesondere eine ausgezeichnete Glätte, als auch einen extrem geringen Leachingeffekt. Die erfindungsgemäßen bioziden Materialien können für die Herstellung medizinischer Artikel oder auch medizinischer Gebrauchsgegenstände jeglicher Art verwendet werden.

Bevorzugt werden die bioziden Materialien der vorliegenden Erfindung für die Herstellung von medizinischen Artikeln, insbesondere ausgewählt aus der Gruppe bestehend aus zentralvenösen Kathetern; peripheren Venenkathetern; Beatmungsschläuche, Stents; Produkte für Anwendung in der Regionalanästhesie, insbesondere Katheter, Kupplungen, Filter; Produkte für die Infusionstherapie, insbesondere Behältnisse, Ports, Überleitungssysteme, Filter; Zubehör wie Konnektoren, Spikes, Ventile, Dreiwegehähne, Spritzen, Leitungen, Zuspritzports; Produkte der Zubereitung, insbesondere Transfersets, Mixsets; urologische Produkte, insbesondere Katheter, Urinmess- und -sammelgeräte; Wunddrainagen; Wundversorgung; chirurgische Nahtmaterialien; Implantationshilfsstoffe sowie Implantate, insbesondere Kunststoffimplantate, beispielsweise Herniennetze, Vliese, Gestricke, Gewirke, Ports, Portkatheter, Gefäßprothesen; Desinfektionsmittel; chirurgisches Einmalinstrumentarium; Thoraxdrainagen; Sonden; Katheter; Gehäuse von medizinischen Geräten, insbesondere Infusionspumpen, Dialysegeräte und Monitore; künstliche Gebisse; Behälter für Flüssigkeiten, insbesondere Kontaktlinsenbehälter.

Weiterhin können die bioziden Materialien der vorliegenden Erfindung auch für die Herstellung von Zubehörteilen von medizinischen Produkten, wie beispielsweise Spritzgussteile und Formteile, verwendet werden. Eine besondere Bedeutung kommt der Verwendung der bioziden Materialien der Erfindung als Additiv in Beschichtungen für chirurgisches Nahtmaterial zu.

Ein weiterhin bevorzugter medizinischer Artikel ist eine Wundauflage.

Insbesondere kann das biozide Material zur Herstellung schlauchförmiger medizinischer Artikel verwendet werden, wie sie insbesondere zuvor definiert wurden. Ein besonders bevorzugter schlauchförmiger medizinischer Artikel ist ein Katheter.

Weitere Merkmale, Einzelheiten und Vorteile der Erfindung ergeben sich aus dem Wortlaut der Ansprüche sowie aus der folgenden Beschreibung von Ausführungsbeispielen.

### Beispiele

### Beispiel 1:

Einfluss des Zusatzes eines erfindungsgemäß einzusetzenden Polyguanidins auf die mechanischen Eigenschaften thermoplastischer Polyurethane (TPU)

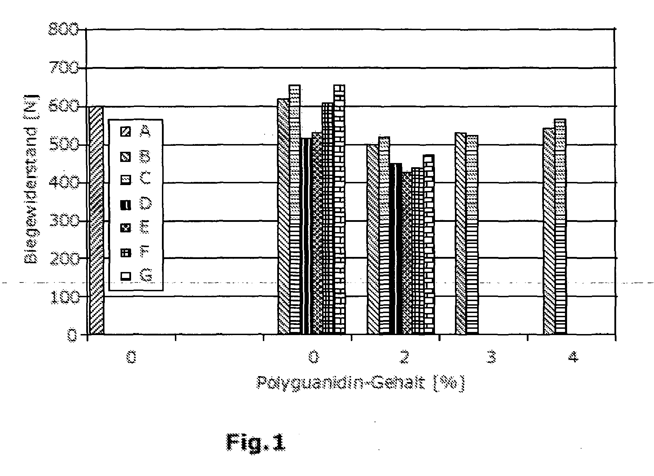

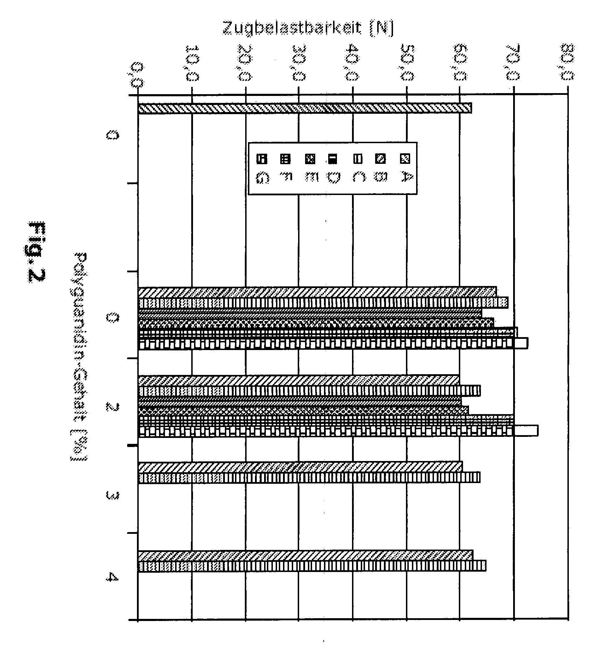

Es werden Muster hergestellt, die thermoplastische Polyurethane verschiedener Hersteller mit jeweils 25 Gew.-% Bariumsulfat und unterschiedlichen Mengen des polymeren Guanidinderivats Poly[2-(2-ethoxy)-ethoxyethyl-guanidiumchlorid] (siehe Fig. 1 und 2) enthalten. Um zusätzlich den Einfluss, der in der Medizintechnik üblichen Sterilisation von Kunststoffen mit Ethylenoxid zu berücksichtigen, wurde jeweils eine Probe mit Ethylenoxid sterilisiert und eine entsprechende Vergleichsprobe nicht sterilisiert. Die Sterilisation mit Ethylenoxid verläuft nach einem dem Fachmann bekannten Verfahren durch Begasung der Proben mit Ethylenoxid bei 40 - 50°C.

**Tabelle 1: Verschiedene TPU's mit BaSO₄**

| Probe | BaSO₄¹ | TPU | Sterilisation mit Ethylenoxid |
|---|---|---|---|
| A | 25 Gew.-% | Pellethane^{®2} | ------- |
| B | 25 Gew.-% | Pellethane^{®} | ------- |
| C | 25 Gew.-% | Pellethane^{®} | ja |
| D | 25 Gew.-% | Elastollan^{®3} | ------- |
| E | 25 Gew.-% | Elastollan^{®} | ja |
| F | 25 Gew.-% | Tecothane^{®4} | ------- |
| G | 25 Gew.-% | Tecothane | ja |

| | | | |
|---|---|---|---|
| ¹ Bariumsulfat mit einer mittleren Korngröße von 0,7 µm ² Pellethane^{®} (thermoplastisches Polyurethan ex Dow Chemicals, USA) ³ Elastollan^{®} (thermoplastisches Polyurethan ex Elastogran GmbH, Deutschland) ⁴ Tecothane^{®} (thermoplastisches Polyurethan ex Thermetics Polymer Products, USA) | | | |

Die Ergebnisse der Biegewiderstandsmessung und der Zugbelastbarkeit sind in den Figuren 1 bzw. 2 dargestellt.

Es zeigt sich, dass der Zusatz des eingesetzten Polyguanidins als Additiv zu Kunststoffen keinen signifikanten Einfluss auf die mechanischen Eigenschaften der Kunststoffe hat.

### Beispiel 2:

### Herstellung eines dreilumigen Katheterschlauchs

Es wird das thermoplastische Polyurethan Pellethane^{®} 2363-90A (Lubrizol Advanced Materials; USA) mit 25 Gew.-% Bariumsulfat mit einer mittleren Korngröße von 0,7 µm und 2 Gew.-% Poly[2-(2-ethoxy)-ethoxyethyl-guanidiniumchlorid] gemischt und anschließend extrudiert. Die Extrusion erfolgte mit dem Extruder Maillefer Typ ED45-30D.

### Beispiel 3 (Vergleichsbeispiel):

Es wird ein Katheterschlauchstück entsprechend Beispiel 2 hergestellt, allerdings ohne Zusatz des Polyguanidins.

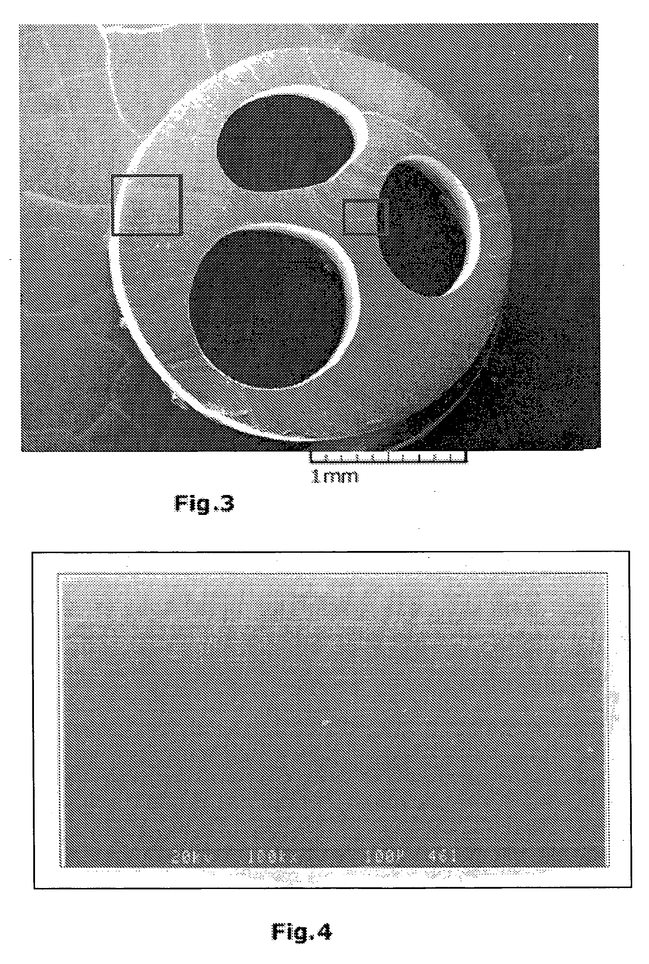

Figur 3 zeigt das gemäß Beispiel 2 hergestellte dreilumige Katheterschlauchstück. Es konnte eine homogene Verteilung des eingesetzten Polyguanidins in dem extrudierten Polymerwerkstoff festgestellt werden.

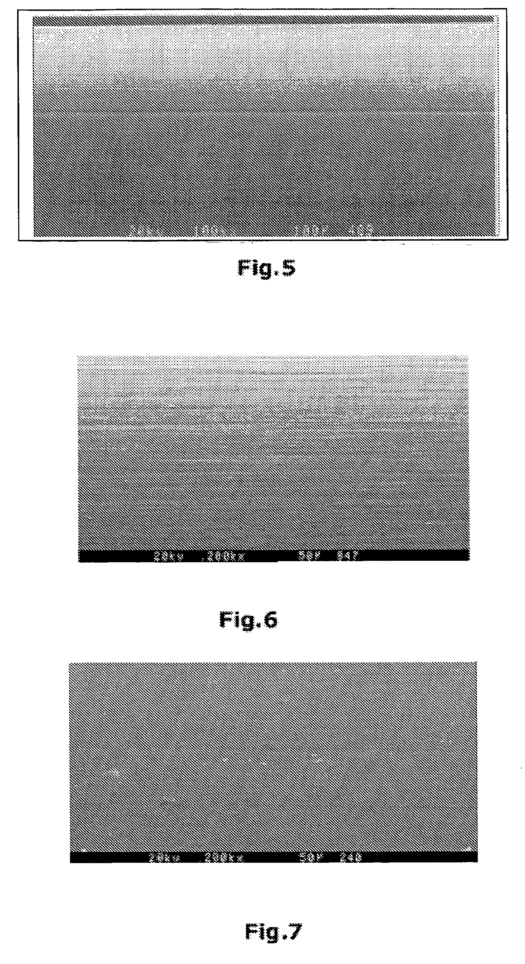

Die gemäß Beispiel 2 und 3 hergestellten Katheterschlauchstücke wurden durch rasterelektronenmikroskopische Aufnahmen untersucht. Figur 4 und 6 zeigen REM-Aufnahmen in unterschiedlicher Auflösung von Katheterschlauchstücken, die gemäß dem Vergleichsbeispiel 3 hergestellt wurden. Figur 5 und 7 zeigen Oberflächenaufnahmen des gemäß Beispiel 2 hergestellten Katheterschlauchstücks.

Es zeigt sich, dass der Zusatz des Polyguanidins zu einer sehr glatten Kunststoffoberfläche führt, was insbesondere wichtig für den Einsatz von Kathetern ist, um die Thrombozytenapposition und Biofilmbildung zu verringern bzw. zu vermeiden.

### Beispiel 4:

### Herstellung eines zentralvenösen Katheters

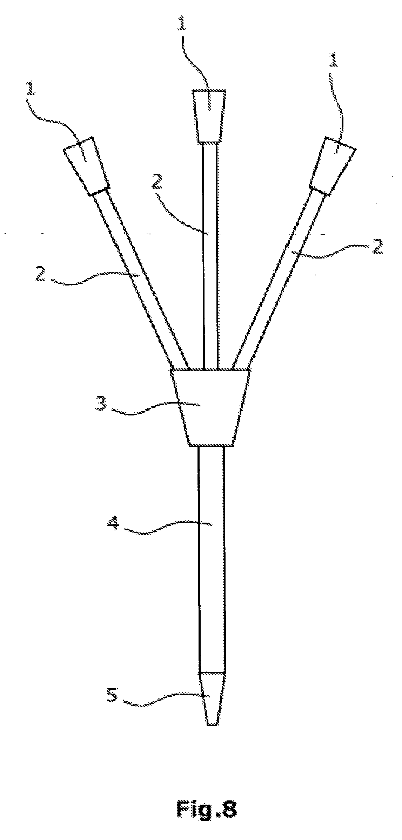

Figur 8 zeigt einen zentralvenösen Katheter mit einem dreilumigen Katheterschlauch (4), Anschluss bzw. Zuleitungen (2), der Kanalverzweigung (3), Anschlussstücken (1) und einer Spitze (5). Die Teile (1) bis (5) können jeweils aus unterschiedlichen Kunststoffen bestehen und diese können wiederum jeweils die erfindungsgemäß zu verwendenden Polyguanidine als Additive aufweisen.

Als Ausgangsmaterial für die Herstellung des Katheters wird ein bereits vorgefertigtes Kunststoffgranulat, welches bereits das erfindungsgemäß einzusetzende polymere Guanidinderivat in der gewünschten Konzentration enthält, eingesetzt.

Die schlauchartigen Komponenten des Katheters (Katheterschlauch (4), Zuleitung (2) und Spitze (5)) werden als Halbzeuge in der Extrusion gefertigt. Die Anlagen zur Extrusion der Compounds (Granulatherstellung) unterscheiden sich nicht von den Anlagen zur Verarbeitung der jeweiligen Grundwerkstoffe. Die Schlauchabschnitte können somit nach den einem Fachmann auf dem Gebiet der Kunststofftechnik bekannten Verfahren hergestellt werden.

Im Einzelnen wird der in Fig. 8 dargestellte zentralvenöse Katheter wie folgt hergestellt:
a) Katheterschlauch (4):
   Ablängen des Schlauchabschnitts, Schlitzen des distalen Endes, ausstanzen der seitlichen Öffnungen des proximalen Endes. Die zur Verarbeitung eingesetzten Anlagen und Prozesse unterscheiden sich nicht von denen zur Verarbeitung von Schlauchabschnitten ohne antimikrobielle Ausstattung. Alle Anlagen und Prozesse entsprechen dem Stand der Technik.
b) Zuleitung (2):
   Ablängen der Schlauchabschnitte. Die zur Verarbeitung eingesetzten Anlagen und Prozesse unterscheiden sich nicht von denen zur Verarbeitung von Schlauchabschnitten ohne antimikrobielle Ausstattung. Alle Anlagen und Prozesse entsprechen dem Stand der Technik.
c) Softspitze (5):
   Ablängen der Schlauchabschnitte. Die zur Verarbeitung eingesetzten Anlagen und Prozesse unterscheiden sich nicht von denen zur Verarbeitung von Schlauchabschnitten ohne antimikrobielle Ausstattung. Alle Anlagen und Prozesse entsprechen dem Stand der Technik.
   Ergebnis dieser Arbeitsschritte sind Schlauchabschnitte deren Geometrie der für das Endprodukt geforderten Geometrie entspricht.
d) Umspritzen der Zuleitungen mit den Ansätzen:
   Die Ansätze werden im Spritzguss gefertigt, in diesem Arbeitsschritt ist das Zusammenfügen der Ansätze mit den Zuleitungen integriert. Die zur Verarbeitung eingesetzten Anlagen und Prozesse unterscheiden sich nicht von denen zur Verarbeitung von Schlauchabschnitten und Granulat ohne antimikrobielle Ausstattung. Alle Anlagen und Prozesse entsprechen dem Stand der Technik.
   Ergebnis des Arbeitsschritts sind Zuleitungen, die dauerhaft mit den Ansätzen zusammengefügt sind.
e) Anformen der Spitze:
   Die Softspitze erhält die endgültige Form durch Thermoformen. In diesem Arbeitsschritt ist das Zusammenfügen von Softspitze und Katheterschlauch integriert. Der Fügeprozess entspricht dabei einem Schweißen von Kunststoffen. Die zur Verarbeitung eingesetzten Anlagen und Prozesse unterscheiden sich nicht von denen zur Verarbeitung von Schlauchabschnitten ohne antimikrobielle Ausstattung. Alle Anlagen und Prozesse entsprechen dem Stand der Technik.
   Ergebnis des Arbeitsschritts ist eine dauerhaft mit dem Katheterschlauch verbundene Softspitze mit der geforderten Form.
f) Umspritzen des Katheterschlauchs und der Zuleitungen mit der Kanaltrennung:
   Die Kanaltrennung wird im Spritzgussverfahren gefertigt. In diesem Arbeitsschritt sind das Zusammenfügen von Katheterschlauch und Kanaltrennung sowie von Zuleitungen und Kanaltrennung integriert. Die zur Verarbeitung eingesetzten Anlagen und Prozesse unterscheiden sich nicht von denen zur Verarbeitung von Schlauchabschnitten und Granulat ohne antimikrobielle Ausstattung. Alle Anlagen und Prozesse entsprechen dem Stand der Technik.
   Ergebnis des Arbeitsschritts ist ein Katheter, der in den Abmessungen und der Gestalt einem zentralnervösen Katheter entspricht.

### Beispiel 5:

Gemäß Beispiel 4 wurden die folgenden schlauchartigen Komponenten eines zentralvenösen Katheters hergestellt:
C1: eine Anschlussleitung aus Pellethane^{®} ohne Polyguanidinadditiv
C2: Katheterschlauch (4) aus Pellethane^{®} 2363-90A mit 25 Gew.-% Bariumsulfat (Korngröße: 0,7 µm) und 2 Gew.-% Poly[2-(2-ethoxy)-ethoxyethyl-guanidiniumchlorid]
C3: wie C2, nur dass 3 Gew.-% Poly[2-(2-ethoxy)-ethoxyethyl-guanidinium-chlorid] zugesetzt wurde
C4: wie C2, nur dass 4 Gew.-% Poly[2-(2-ethoxy)-ethoxyethyl-guanidinium-chlorid] zugesetzt wurde
C5: wie C2, nur dass statt dem Pellethane^{®} Elastolan^{®} eingesetzt wurde
C6: Softspitze aus Tecothane^{®}, 25 Gew.-% Bariumsulfat (Korngröße: 0,7 µm) und 2 Gew.-% Poly[2-(2-ethoxy)-ethoxyethyl-guanidiniumchlorid].

Die schlauchartigen Komponenten C1 bis C6 wurden auf ihre antimikrobielle Wirksamkeit im Rahmen eines Proliferationstests (Bechert et al. "A new method for screening anti-effective biomaterials"; Nature Medicine 2000, 6(9): 1053-1056) auf verschiedene Bakterienstämme getestet. Die Proben werden als "antimikrobiell" bezeichnet, wenn sie eine Reduktion der Vermehrungsfähigkeit der jeweiligen Keime um mindestens 3 log-Stufen zeigen.

Tabelle 2 zeigt das Ergebnis.

**Tabelle 2:**

| Antimikrobielle Wirksamkeit medizinischer Artikel gegen verschiedene Bakterienstämme | | | | | |
|---|---|---|---|---|---|
| Katheterkomponente | Staphylococcus epidermis | Staphylococcus aureus | Staphylococcus aureus MRSA | Candida albicans | E. coli |
| C1 | nicht antimikrobiell | nicht antimikrobiell | nicht antimikrobiell | nicht antimikrobiell | nicht antimikrobiell |
| C2 | antimikrobiell | antimikrobiell | antimikrobiell | antimikrobiell | nicht antimikrobiell |
| C3 | antimikrobiell | antimikrobiell | antimikrobiell | antimikrobiell | antimikrobiell |
| C4 | antimikrobiell | antimikrobiell | antimikrobiell | antimikrobiell | antimikrobiell |
| C5 | antimikrobiell | antimikrobiell | antimikrobiell | antimikrobiell | antimikrobiell |
| C6 | antimikrobiell | antimikrobiell | antimikrobiell | antimikrobiell | antimikrobiell |

Wie der Tabelle 2 zu entnehmen ist, sind die mit den erfindungsgemäß zu verwendenden Polyguanidinen ausgerüsteten schlauchartigen Katheterkomponenten hochwirksam gegen die Stämme Staphylococcus epidermidis, Staphylococcus aureus, Staphylococcus aureus MRSA, E. coli sowie Candida albicans.

Die Proben C2 und C4 wurden darüber hinaus einem Hämolysetest und einem Leaching-Test mit Ethanol und Wasser unterzogen. Der Leaching-Test wurde durchgeführt, indem das Testmaterial über einen Zeitraum von 24 h bei einer Temperatur von 37 °C mit destilliertem Wasser, das 5 % Ethanol enthält, extrahiert wurde. Das Oberfläche-zu-Volumen-Verhältnis der Testmaterialien betrug 3 cm²/ml. Der Extrakt wird anschließend auf Polyguanidine analysiert. Hierzu werden 0,2 g Kaliumhexacyanoferrat und 0,2 g Natriumnitroprussid in 4 ml einer 6N Natriumhydroxidlösung gegeben und die so erhaltene Lösung auf 100 ml entionisiertes Wasser aufgefüllt. 1 ml des Extrakts wird getrocknet und anschließend in 1 ml entionisiertem Wasser aufgelöst. Anschließend werden 4 ml des zuvor hergestellten Nachweisreagenzes hinzugefügt, die Lösung wird anschließend mittels UV/VIS-Spektroskopie quantitativ analysiert.

Der Hämolysetest zeigte, dass keine Substanzen in hämolytischen Konzentrationen freigesetzt werden. Der Leaching-Test zeigte darüber hinaus, dass Extraktion mit Ethanol/Wasser zu keiner Detektion des eingesetzten Polyguanidins führte.

### Beispiel 6:

Es wurden für den Introcan^{®} Safety Katheter der Firma B. Braun, Melsungen sowohl der Katheter als auch das Gehäuse mit dem erfindungsgemäß einzusetzenden Polyguanidin ausgerüstet.

Es wurden hierzu die folgenden medizinischen Artikel hergestellt:
D1: Tecoflex^{®} Katheter aus Polyurethan sowohl mit als auch ohne Silikonölbeschichtung und ohne Polyguanidinadditiv
D2: Tecoflex^{®} Katheter aus Polyurethan mit 2 Gew.-% Poly[2-(2-ethoxy)-ethoxyethyl-guanidiniumchlorid] und sowohl mit als auch ohne zusätzlicher Silikonölbeschichtung
D3: Tecoflex^{®} Katheter aus Polyurethan mit 4 Gew.-% Poly[2-(2-ethoxy)-ethoxyethyl-guanidiniumchlorid] und sowohl mit als auch ohne zusätzlicher Silikonölbeschichtung
D4: Introcan^{®} Safety Gehäuse aus Polypropylen ohne Polyguanidinadditiv
D5: Introcan^{®} Safety Polypropylengehäuse mit 2 Gew.-% Poly[2-(2-ethoxy)-ethoxyethyl-guanidiniumchlorid]
D6: Introcan^{®} Safety Polypropylengehäuse mit 4 Gew.-% Poly[2-(2-ethoxy)-ethoxyethyl-guanidiniumchlorid].

Die antimikrobielle Wirksamkeit der medizinischen Artikel wurde anhand eines Proliferationstests (Bechert et al. "A new method for screening anti-effective biomaterials"; Nature Medicine 2000, 6(9): 1053-1056) in Tabelle 3 zusammengefasst. Darüber hinaus zeigte das Beispiel D2 keinen Leaching-Effekt (Testbeschreibung siehe Beispiel 2).

**Tabelle 3:**

| Antimikrobielle Wirksamkeit medizinischer Artikel gegen verschiedene Bakterienstämme | | | |
|---|---|---|---|
| Probe | Staph. epidermis | E. coli | Candida albicans |
| D1 | nicht antimikrobiell | nicht antimikrobiell | nicht antimikrobiell |
| D2 | antimikrobiell | antimikrobiell | antimikrobiell |
| D3 | antimikrobiell | antimikrobiell | antimikrobiell |
| D4 | nicht antimikrobiell | nicht antimikrobiell | nicht antimikrobiell |
| D5 | antimikrobiell | antimikrobiell | antimikrobiell |
| D6 | antimikrobiell | antimikrobiell | antimikrobiell |

## Patentansprüche

1. Verwendung wenigstens eines polymeren Guanidinderivats mit biozider Wirkung, welches erhältlich ist durch Polykondensation eines Guanidin-Säureadditionssalzes mit einem Amingemisch, das wenigstens ein Diamin enthält, welches ausgewählt ist aus der Gruppe bestehend aus Aklylendiamin und Oxyalkylendiamin, als Additiv in einer Zusammensetzung für schlauchförmige medizinische Artikel.

2. Verwendung gemäß Anspruch 1, **dadurch gekennzeichnet, dass** das Amingemisch eine erste Komponente und wenigstens eine zweite Komponente enthält, wobei
- die erste Komponente ein Diamin ist, ausgewählt aus der Gruppe bestehend aus Alkylendiamin und Oxyalkylendiamin, und wobei
- die zweite Komponente ein Diamin ist, das ausgewählt aus der Gruppe bestehend aus Alkylendiamin und Oxyalkylendiamin, und
wobei die erste Komponente von der zweiten Komponente verschieden ist.

3. Verwendung gemäß Anspruch 2, **dadurch gekennzeichnet, dass** die erste Komponente Alkylendiamin und die zweite Komponente ein Oxyalkylendiamin ist.

4. Verwendung gemäß Anspruch 3, **dadurch gekennzeichnet, dass** das Alkylendiamin Hexamethylendiamin und das Oxyalkylendiamin Triethylenglykoldiamin ist.

5. Verwendung gemäß mindestens einem der Ansprüche 2 oder 3, **dadurch gekennzeichnet, dass** die erste Komponente und die zweite Komponente in einem Molverhältnis von 4:1 bis 1:4, vorzugsweise 2:1 bis 1:2 vorliegt.

6. Verwendung gemäß mindestens einem der Ansprüche 1 bis 5, **dadurch gekennzeichnet, dass** das polymere Guanidinderivat ein mittleres Molekulargewicht im Bereich von 500 bis 7000, insbesondere von 1000 bis 5000 aufweist.

7. Verwendung gemäß mindestens einem der Ansprüche 1 bis 6, **dadurch gekennzeichnet, dass** das polymere Guanidinderivat in einer Menge von maximal 10,0 Gew.-%, insbesondere von 0,01 bis 6 Gew.-% und im Speziellen in einer Menge von 1,0 bis 4,0 Gew.-%, bezogen auf die Zusammensetzung für den medizinischen Artikel, vorliegt.

8. Verwendung gemäß mindestens einem der Ansprüche 1 bis 7, **dadurch gekennzeichnet, dass** die Zusammensetzung weiterhin Kunststoffe, insbesondere thermoplastische Polymere, vorzugsweise ausgewählt aus Polyurethan, Polyolefin, Polyvinylchlorid, Polycarbonat, Polystyrol, Polyethersulfon, Silikon und Polyamid, aufweist.

9. Verwendung gemäß Anspruch 8, **dadurch gekennzeichnet, dass** das polymere Guanidinderivat kovalent mit dem Kunststoff verknüpft ist.

10. Verwendung gemäß mindestens einem der Ansprüche 1 bis 9, **dadurch gekennzeichnet dass** der medizinische Artikel insbesondere ausgewählt aus der Gruppe bestehend aus Katheter; zentralvenösen Kathetern; peripheren Venenkathetern; Beatmungsschläuche; Stents; Kuppelungen; Ports; Überleitungssysteme; Konnektoren, Spikes, Ventile, Dreiwegehähne, Spritzen, Leitungen, Zuspritzports; Wunddrainage; Thoraxdrainagen und Sonden.

11. Verfahren zur Herstellung eines schlauchförmigen medizinischen Artikels, umfassend die folgenden Schritte:
a) Zusammenführen und Vermischen eines polymeren Guanidinderivats mit biozider Wirkung, welches erhältlich ist durch Polykondensation eines Guanidin-Säureadditionssalzes mit einem Amingemisch, das wenigstens ein Diamin enthält, welches ausgewählt ist aus der Gruppe bestehend aus Aklylendiamin und Oxyalkylendiamin, mit mindestens einem Kunststoff, vorzugsweise einem thermoplastischen Polymer,
b) Einsetzen des unter a) erhaltenen Gemisches in einem oder mehreren Formgebungsverfahren unter Ausbildung eines medizinischen Artikels.

12. Verfahren gemäß Anspruch 11, **dadurch gekennzeichnet, dass** das Vermischen in Schritt a) durch Schmelzkneten, vorzugsweise bei Temperaturen oberhalb von 100°C, weiter bevorzugt oberhalb von 150°C, erfolgt.

13. Schlauchförmiger medizinischer Artikel umfassend ein polymeres Guanidinderivat mit biozider Wirkung, welches erhältlich ist durch Polykondensation eines Guanidin-Säureadditionssalzes mit einem Amingemisch, das wenigstens ein Diamin enthält, welches ausgewählt ist aus der Gruppe bestehend aus Aklylendiamin und Oxyalkylendiamin, sowie mindestens einen Kunststoff, insbesondere ein thermoplastisches Polymer.

14. Schlauchförmiger medizinischer Artikel gemäß Anspruch 13, **dadurch gekennzeichnet, dass** das polymere Guanidinderivat mit dem Kunststoff kovalent verknüpft ist.

15. Schlauchförmiger medizinischer Artikel gemäß Anspruch 13 oder 14, **dadurch gekennzeichnet, dass** es ein Katheter ist.

## Claims

1. Use of at least one polymeric guanidine derivative having biocidal activity that is obtainable by the polycondensation of a guanidine acid addition salt with a mixture of amines containing at least one diamine selected from the group consisting of alkylene diamine and oxyalkylene diamine, as an additive in a composition for tubular medical articles.

2. The use according to claim 1, **characterized in that** said mixture of amines contains a first component and at least one second component, wherein
- the first component is a diamine selected from the group consisting of alkylene diamine and oxyalkylene diamine, and wherein
- the second component is a diamine selected from the group consisting of alkylene diamine and oxyalkylene diamine, and
the first component is different from the second component.

3. The use according to claim 2, **characterized in that** said first component is alkylene diamine and said second component is an oxyalkylene diamine.

4. The use according to claim 3, **characterized in that** said alkylene diamine is hexamethylene diamine and said oxyalkylene diamine is triethylene glycol diamine.

5. The use according to at least either of claims 2 or 3, **characterized in that** said first component and said second component are present in a molar ratio of from 4:1 to 1:4, preferably from 2:1 to 1:2.

6. The use according to at least one of claims 1 to 5, **characterized in that** said polymeric guanidine derivative has an average molecular weight within a range of from 500 to 7000, especially from 1000 to 5000.

7. The use according to at least one of claims 1 to 6, **characterized in that** said polymeric guanidine derivative is present in an amount of at most 10.0% by weight, especially from 0.01 to 6% by weight, and especially in an amount of from 1.0 to 4.0% by weight, respectively based on the composition for the medical article.

8. The use according to at least one of claims 1 to 7, **characterized in that** said composition further includes plastic materials, especially thermoplastic polymers, preferably selected from polyurethane, polyolefin, polyvinyl chloride, polycarbonate, polystyrene, polyethersulfone, silicone and polyamide.

9. The use according to claim 8, **characterized in that** said polymeric guanidine derivative is covalently bonded to said plastic material.

10. The use according to at least one of claims 1 to 9, **characterized in that** said medical article is selected, in particular, from the group consisting of catheters, central venous catheters, peripheral venous catheters, breathing tubes, stents, couplings, ports, conduit systems, connectors, spikes, valves, three-way stopcocks, syringes, conduits, injection ports, wound drains, thoracic drains and probes.

11. A process for preparing a tubular medical article, comprising the following steps:
a) combining and mixing a polymeric guanidine derivative having biocidal activity that is obtainable by the polycondensation of a guanidine acid addition salt with a mixture of amines containing at least one diamine selected from the group consisting of alkylene diamine and oxyalkylene diamine, with at least one plastic material, preferably a thermoplastic polymer;
b) subjecting the mixture obtained under a) to one or more shaping methods to form a medical article.

12. The process according to claim 11, **characterized in that** said mixing in step a) is effected by melt-kneading, preferably at temperatures above 100 °C, more preferably above 150 °C.

13. A tubular medical article comprising a polymeric guanidine derivative having biocidal activity that is obtainable by the polycondensation of a guanidine acid addition salt with a mixture of amines containing at least one diamine selected from the group consisting of alkylene diamine and oxyalkylene diamine, and at least one plastic material, preferably a thermoplastic polymer.

14. The tubular medical article according to claim 13, **characterized in that** the polymeric guanidine derivative is covalently bonded to the plastic material.

15. The tubular medical article according to claim 13 or 14, **characterized by** being a catheter.

## Revendications

1. Utilisation d'au moins un dérivé polymère de guanidine à effet biocide qui peut être obtenu par la polycondensation d'un sel d'addition acide de guanidine avec un mélange d'amines contenant au moins une diamine choisie dans le groupe comprenant alkylène diamine et oxyalkylène diamine, en tant qu'additif dans une composition destinée à des articles médicaux tubulaires.

2. Utilisation selon la revendication 1, **caractérisée en ce que** ledit mélange d'amines contient un premier composant et au moins un second composant, dans laquelle
- ledit premier composant est une diamine choisie dans le groupe comprenant alkylène diamine et oxyalkylène diamine, et dans laquelle
- ledit second composant est une diamine choisie dans le groupe comprenant alkylène diamine et oxyalkylène diamine, et
dans laquelle ledit premier composant est différent dudit second composant.

3. Utilisation selon la revendication 2, **caractérisée en ce que** ledit premier composant est une alkylène diamine et ledit second composant est une oxyalkylène diamine.

4. Utilisation selon la revendication 3, **caractérisée en ce que** ladite alkylène diamine est la hexaméthylène diamine, et ladite oxyalkylène diamine est la triéthylène glycol diamine.

5. Utilisation selon au moins une des revendications 2 ou 3, **caractérisée en ce que** ledit premier composant et ledit second composant sont présents dans un rapport molaire de 4:1 à 1:4, de préférence de 2:1 à 1:2.

6. Utilisation selon au moins une des revendications 1 à 5, **caractérisée en ce que** ledit dérivé polymère de guanidine présente un poids moléculaire moyen compris entre 500 et 7000, notamment entre 1000 et 5000.

7. Utilisation selon au moins une des revendications 1 à 6, **caractérisée en ce que** ledit dérivé polymère de guanidine est présent en une quantité d'au plus 10,0 % en poids, notamment de 0,01 à 6 % en poids, et spécifiquement en une quantité de 1,0 à 4,0 % en poids, par rapport à ladite composition destinée audit article médical.

8. Utilisation selon au moins une des revendications 1 à 7, **caractérisée en ce que** ladite composition comprend en outre des matières plastiques, notamment des polymères thermoplastiques, de préférence choisies parmi polyuréthane, polyoléfine, polychlorure de vinyle, polycarbonate, polystyrène, polyéthersulfone, silicone et polyamide.

9. Utilisation selon la revendication 8, **caractérisée en ce que** ledit dérivé polymère de guanidine est lié de manière covalente à ladite matière plastique.

10. Utilisation selon au moins une des revendications 1 à 9, **caractérisée en ce que** ledit article médical est notamment choisi dans le groupe consistant en des cathéters, des cathéters veineux central, des cathéters veineux périphérique, des tubes respiratoires, des endoprothèses, des couplages, des pièces de connexion, des systèmes de conduite, des connecteurs, des crampons, des soupapes, des robinets à trois voies, des seringues, des conduits, des ouvertures à injection, des drains de plaie, des drains thoraciques et des sondes.

11. Procédé pour la production d'un article médical tubulaire, comprenant les étapes consistant à :
a) combiner et mélanger un dérivé polymère de guanidine à effet biocide qui peut être obtenu par la polycondensation d'un sel d'addition acide de guanidine avec un mélange d'amines contenant au moins une diamine choisie dans le groupe comprenant alkylène diamine et oxyalkylène diamine, avec au moins une matière plastique, de préférence un polymère thermoplastique ;
b) soumettre le mélange obtenu dans l'étape a) dans un ou plusieurs procédés de façonnage pour former un article médical.

12. Procédé selon la revendication 11, **caractérisé en ce que** le processus de mélange dans l'étape a) est effectué par malaxage à l'état fondu, de préférence à des températures supérieures à 100 °C, mieux encore supérieures à 150 °C.

13. Article médical tubulaire, comprenant un dérivé polymère de guanidine à effet biocide qui peut être obtenu par la polycondensation d'un sel d'addition acide de guanidine avec un mélange d'amines contenant au moins une diamine choisie dans le groupe comprenant alkylène diamine et oxyalkylène diamine, ainsi qu'au moins une matière plastique, notamment un polymère thermoplastique.

14. Article médical tubulaire selon la revendication 13, **caractérisé en ce que** ledit dérivé polymère de guanidine est lié de manière covalente à ladite matière plastique.

15. Article médical tubulaire selon la revendication 13 ou 14, **caractérisé en ce qu'**il s'agit d'un cathéter.
